# EUROPEAN PATENT APPLICATION

(11) **EP 4 450 632 A2**
(43) Date of publication of application: **23.10.2024**
(21) Application number: 24171413.8
(22) Date of filing: 19.04.2024
(51) Int. Cl.: C12P 7/625, C08L 67/04

(54) **METHOD FOR THE PRODUCTION OF POLYHYDROXYALKANOATES USING PHOTOTROPHIC MIXED CULTURES AND CO2 AS CARBON SOURCE**

(30) Priority: 20.04.2023 PT 2023118611
(71) Applicant: UNIVERSIDADE NOVA DE LISBOA, 1099-085 Lisboa (PT)
(72) Inventor: CORREIA FRECHES, ANDRÉ, 2829-516 CAPARICA (PT); COSTA FRADINHO, JOANA, 2829-516 CAPARICA (PT); RODA ALMEIDA, JULIANA, 2829-516 CAPARICA (PT); CARVALHO FERNANDES MIRANDA REIS, MARIA D'ASCENSÃO, CAPARICA (PT)
(74) Representative: Pereira da Cruz, Joao

(57) **Abstract**

Methods for production of polyhydroxyalkanoate (PHA) are disclosed. The present disclosure is directed to a method for production of PHA, the method comprising: adding to a microbial culture comprising at least one phototrophic microorganism, a feedstock comprising at least one inorganic carbon compound selected from carbon dioxide, carbonic acid, carbonate and bicarbonate and at least one electron donor, wherein the adding is performed continuously or discontinuously, under non-sterile anaerobic conditions and in the presence of infra-red radiation.

## Description

### FIELD OF THE INVENTION

The present disclosure, in some embodiments thereof, relates to biopolymer production.

### BACKGROUND OF THE INVENTION

Conventional plastics are essential materials but most of them are nondegradable and accumulate in the environment causing serious pollution problems. As an alternative, PHA is a biodegradable polymer naturally synthesized by many bacteria and presents properties similar to traditional plastics. In the last years, PHA became more economically competitive through its production with mixed microbial cultures fed with low-cost feedstocks (e.g., wastewater). In line with this, several PHA producing systems have been developed including a recent technology that uses phototrophic mixed cultures (PMC). Results indicated that phototrophic bacteria are fully capable of producing PHA from real organic wastes.

On the other hand, global warming is pressing scientists to find new routes to fixate CO₂. In 2013, CO₂ levels surpassed 400 ppm for the first time in recorded history. Climate change related to CO₂ levels is already happening, with extreme atmospheric events being recorded more often than ever. This recent relentless rise in CO₂ needs to be tackled and the transformation of CO₂ into biodegradable biopolymers will both contribute to the reduction of air and plastic pollution while producing novel added-value products.

Patent WO2019193518A2 describes methods, systems, and compositions for the production of bioplastic from a gaseous substrate containing a carbon source by a two-stage fermentation process.

Patent WO2017085653A1 describes a method for producing polyhydroxyallkanoates (PHA) using a mixed culture of selected photosynthetic organisms in a bioreactor preferably operated with illumination in non-sterile and anaerobic conditions and in the presence of at least one carbon source.

Therefore, there is a need for new technologies beyond the usual utilization of organic feedstocks and advance to the new research field of CO₂-based plastics. In particular, technologies able to transform directly inorganic carbon into biopolymers in a single step process.

### SUMMARY OF THE INVENTION

In one aspect of the disclosure, there is provided a method for production of polyhydroxyalkanoate (PHA), the method comprising: a) adding to a microbial culture comprising at least one phototrophic microorganism, a feedstock comprising at least one inorganic carbon compound and at least one electron donor, wherein the adding is performed continuously or discontinuously, under non-sterile anaerobic conditions and in the presence of infra-red radiation, thereby obtaining a mixture comprising PHA; and b) separating at least a first fraction from the mixture.

In some embodiments, the step a) is performed in cycles alternating between a cycle in presence of infra-red radiation, preferably for at least one period of time from 1 hour (h) to 30 days, more preferably from 6 h to 12h, and a cycle devoid of light, preferably for at least one period of time from 1minute (min) to 48 h, more preferably from 4 h to 8 h.

In some embodiments, the electron donor comprises at least one reduced sulfur compound, preferably comprising at least one sulfide.

In some embodiments, the electron donor comprises hydrogen.

In some embodiments, the feedstock is a gas stream, a liquid stream, or both.

In some embodiments, the inorganic carbon compound comprises carbon dioxide, carbonic acid, carbonate, bicarbonate salt or any combination thereof.

In some embodiments, the feedstock further comprises at least one nutrient, preferably selected from the list: nitrogen, phosphorus, potassium, magnesium, calcium, iron, sodium, cobalt, zinc, manganese, boron, copper, chlorine, nickel, selenium, iron, molybdenum, bromine, iodine, vitamins, or any combination thereof.

In some embodiments, the microbial culture comprises bacteria, preferably an anoxygenic phototrophic bacteria.

In some embodiments, the adding comprises permanent feed and/or intermittent feed of the at least one inorganic carbon compound, at least one electron donor, or both.

In some embodiments, the method further comprises a step of maintaining the at least a first fraction under non-sterile anaerobic conditions, in the presence of infra-red radiation, devoid of light, or alternating between both.

In some embodiments, the method further comprises adding feedstock continuously or discontinuously to the first fraction.

In some embodiments, the method further comprises a step of separating from the first fraction, a solid fraction comprising the PHA.

In some embodiments, the method further comprises isolating, purifying, or both, the PHA from the solid fraction.

In another aspect of the disclosure, there is provided a composition comprising polyhydroxyalkanoate (PHA), obtained by the method according to the present disclosure.

In another aspect of the disclosure, there is provided a use of the composition according to the present disclosure, in animal feed, biocontrol agent, bioplastic, food packaging, drinks packaging, pharmaceuticals packaging, medical devices, tissue engineering, sutures, fibers and clothing.

Unless otherwise defined, all technical and/or scientific terms used herein have the same meaning as commonly understood by one of ordinary skill in the art to which the disclosure pertains. Although methods and materials similar or equivalent to those described herein can be used in the practice or testing of embodiments of the disclosure, exemplary methods and/or materials are described below. In case of conflict, the patent specification, including definitions, will control. In addition, the materials, methods, and examples are illustrative only and are not intended to be necessarily limiting.

Further embodiments and the full scope of applicability of the present disclosure will become apparent from the detailed description given hereinafter. However, it should be understood that the detailed description and specific examples, while indicating preferred embodiments of the disclosure, are given by way of illustration only, since various changes and modifications within the spirit and scope of the disclosure will become apparent to those skilled in the art from this detailed description.

### BRIEF DESCRIPTION OF THE DRAWINGS

Some embodiments of the disclosure are herein described, by way of example only, with reference to the accompanying drawings. With specific reference now to the drawings in detail, it is stressed that the particulars shown are by way of example and for purposes of illustrative discussion of embodiments of the disclosure. In this regard, the description taken with the drawings makes apparent to those skilled in the art how embodiments of the disclosure may be practiced.
Figure 1 is a scheme of an experimental setup of phototrophic mixed cultures (PMC) selection in sequencing batch reactor (SBR): SBR (1); magnetic stirrer (2); 3 lamps (3); acid and basic solutions for pH control (4 and 5); bags with argon to fill the headspace (6); carbon and sulfide sources (7 and 8); mineral medium (9); purge (10); A - headspace outlet; B - sensor to measure temperature, pH and oxidation reduction potential (ORP);
Figure 2 is a diagram presenting the different stages of photobioreactor operation;
Figure 3 is a graph presenting high sulfide and low sulfide approaches;
Figure 4 is a graph presenting 24 hours (h) cycle and 12h cycle operation;
Figures 5A-C are graphs of ORP variation (Figure 5A), sulfides concentration overtime (Figure 5B) and inorganic carbon concentration overtime (Figure 5C);
Figure 6 is a bar graph presenting light intensity tests; and
Figure 7 are photos of microbial species with fluorescence using Nile Blue staining, after 6h in dark conditions.

### DETAILED DESCRIPTION OF THE INVENTION

According to some embodiments, the present disclosure provides a method for production of polyhydroxyalkanoate (PHA). The present disclosure provides an autotrophic process for capturing and transforming inorganic carbon into a biopolymer.

According to some embodiments, the method comprises a step a) contacting a microbial culture comprising at least one phototrophic microorganism, with a feedstock comprising at least one inorganic carbon compound and at least one electron donor, under non-sterile anaerobic conditions and in the presence of infra-red radiation, thereby obtaining a mixture comprising PHA.

According to some embodiments, the method comprises a step a) adding to a microbial culture comprising at least one phototrophic microorganism, a feedstock comprising at least one inorganic carbon compound and at least one electron donor. In some embodiments, the adding is performed continuously or discontinuously, under non-sterile anaerobic conditions and in the presence of infra-red radiation. In some embodiments, step a) is performed under stirring. In some embodiments, step a) is performed under continuous stirring.

In some embodiments, the at least one inorganic compound and the at least one electron donor are added together. In some embodiments, the at least one inorganic compound and the at least one electron donor are added separately.

In some embodiments, the at least one inorganic carbon compound is added in low concentrations. In some embodiments, the at least one inorganic carbon compound is added in high concentrations. In some embodiments, the at least one inorganic carbon compound is added in a concentration from 0.1 mM to 50 mM. In some embodiments, the at least one inorganic carbon compound is added in a concentration from 1 mM to 20 mM.

In some embodiments, at least one electron donor is added in a concentration from 0.01 to 10 mM. In some embodiments, at least one electron donor is added in a concentration from 0.1 to 10 mM. In some embodiments, at least one electron donor is added in a concentration from 1 to 10 mM. In some embodiments, at least one electron donor is added in a concentration from 2 to 8 mM. In some embodiments, at least one electron donor is added in a concentration of less than 10 mM. In some embodiments, at least one electron donor is added in a concentration of less than 5 mM.

In some embodiments, the electron donor is added at a loading rate from 0.01 to 5 mM S d⁻¹. In some embodiments, the electron donor is added at a loading rate from 0.05 to 5 mM S d⁻¹. In some embodiments, the electron donor is added at a loading rate from 0.1 to 5 mM S d⁻¹. In some embodiments, the electron donor is added at a loading rate from 0. 1 to 3 mM S d⁻¹.

The present disclosure is based, in part, on the finding that the conditions as described hereinabove promote the enrichment of the microbial culture in PHA accumulating anoxygenic phototrophic organisms, thereby promoting the formation of PHA. The present disclosure is based, in part, on the finding that a process as described herein allows the direct transformation of inorganic carbon into a biopolymer, in a single step. The technical effect associated with the direct conversion of carbon is a greater efficiency in the transformation of carbon, lower operating costs, and less probability of disruptions in the microbial culture.

According to the present disclosure, the feedstock is added to the microbial culture, continuously or discontinuously. In some embodiments, each component of the feedstock is added to the microbial culture continuously or discontinuously.

The term "continuously" as described herein, refers to a stream of a component/compound that is constantly added to the microbial culture, without interruptions. The term "discontinuously" as described herein, refers to a stream of a component/compound that is added to the microbial culture during a certain period of time (a cycle), and in different time intervals, for at least one time interval. In some embodiments according to the present disclosure, the term "discontinuously" and "intermittent" are used interchangeably.

In some embodiments, adding comprises permanent feed and/or intermittent feed of the at least one inorganic carbon compound, at least one electron donor, or both.

In some embodiments, the method comprises permanently feed the at least one inorganic compound to the microbial culture. In some embodiments, the method comprises feed the at least one electron donor to the microbial culture in intermittent (feast/famine) cycles.

According to the present disclosure, the use of infra-red radiation and anaerobic conditions, leads to the selection of a microbial community comprising anoxygenic phototrophic microorganisms, suitable and with higher efficiency for PHA storage.

According to the present disclosure, the conditions used in step a) as described hereinabove, enriches the microbial culture, in a mixed culture rich in PHA-accumulating photosynthetic organisms that produce PHA by microbial biosynthesis.

In some embodiments, the at least one phototrophic microorganism comprises bacteria, preferably an anoxygenic phototrophic bacteria. In some embodiments, the at least one phototrophic microorganism comprises a mixed microbial culture.

Anoxygenic and phototrophic species are well known in the art and would become apparent to those skilled in the art. Non-limiting examples of such species include *Chloroflexus aggregans, Chloroflexus aurantiacus, Chloroflexus islandicus, Rhodopseudomonas palustris, Rhodobacter sphaeroids, Rhodospirillum rubrum, Lamprobacter modestohalophilus, Chromatium okenii* and *Thiocapsa roseopersicina.*

According to the present disclosure, a microbial culture as described herein is a mixed microbial culture. According to the present disclosure, a microbial culture as described herein is a microbial biomass.

The term "mixed microbial culture" as described herein, refers to a community comprising different organisms and different species that include bacteria, fungi, microalgae, or any combination thereof. The term mixed microbial culture also includes enriched microbial communities, preferably bacterial communities. According to the present disclosure, an enriched microbial community is obtained, by imposing favorable conditions as described herein, to the growth of organisms that positively affect the production of PHA and unfavorable to the growth of organisms that negatively affect PHA production.

According to the present disclosure, microbial cultures obtained from biomass from different sources can be used. In the case of photosynthetic bacteria, they can be found in environments that allow the availability of light. However, due to their metabolic diversity, they may also be found in dark areas where oxygen or fermentable compounds are available. As such, the presence of these bacteria is frequent in shallow pond and pond sediments, wet soils and swamps, and can also be found in activated sludge, landfills, mine soils and saline environments. The preferred source for obtaining these organisms includes environments subject to periodic stresses such as carbon starvation, essential nutrients or oxygen. The occurrence of these limitations is expected to select bacteria that accumulate carbon reserves in response to stressful situations. It should be understood that organisms isolated from different environments may have different yields and PHA production capacities.

According to some embodiments, the microbial culture (phototrophic mixed microbial culture) uses inorganic compounds (including inorganic carbon) from the feedstock to grow and accumulate internal polymers, including glycogen and polyhydroxyalkanoates (PHA).

According to the present disclosure, "inorganic carbon compound" refers to any inorganic compound comprising CO₂, carbonate, bicarbonate salts, or any combination thereof. In some embodiments, the inorganic carbon compound comprises carbon dioxide, carbonic acid, carbonate, bicarbonate salt or any combination thereof.

According to some embodiments, the method comprises a step b) separating at least a first fraction from the mixture.

In some embodiments, between cycles (step a), at least a portion of the volume of the mixture (step b) is withdrawn/separated from the mixture. The technical effect associated is the fact that under these conditions, the mixed culture of phototrophic organisms [phototrophic mixed culture (PMC)] is capable of internally accumulating PHA to higher levels according to the dominant population of the system. According to the present disclosure, step b) can be performed, at different time points of the process and for at least 1 time, at least 2 times, at least 3 times, at least 5 times, at least 10 times, at least 50 times, at least 100 times, at least 200 times or at least 500 times. It should be understood that the number of times that step b) is performed or repeated, can be adapted and depends on the scale, amount of feedstock added to the microbial mixture, period of time of step a), level of PHA-accumulating photosynthetic organisms in the microbial mixture, or any combination thereof.

In some embodiments, step b) is performed continuously or discontinuously, according to the amount of feedstock added to the microbial mixture.

According to some embodiments, a method as described herein, is performed in sequential cycles of step a) and step b). The present disclosure is based, in part, on the finding that step a) and step b) allows for a selective growth of a mixture of microorganisms that increase the production of PHA.

According to some embodiments, a method as described herein, uses inorganic carbon as the only carbon source for the growth of a mixed culture of phototrophic organisms, for the for production of PHA. In some embodiments, the method is performed in an illuminated reactor, with no aeration, and in non-sterile conditions.

In some embodiments, illumination is with infra-red radiation. The present disclosure is based, in part, on the finding that the use of infra-red radiation promotes the elimination of phototrophic oxygen producers, such as microalgae and cyanobacteria which require visible light to grow. The technical effect associated with the elimination of microalgae and cyanobacteria is the fact that microalgae do not produce PHA, and reduces oxygen production which is not desirable since the conditions must be anaerobic. In some embodiments, the anaerobic conditions are essential to promote high PHA storage and allow the anaerobic internal conversion of glycogen into PHA. According to the present disclosure, PHA is directly produced, and obtained by conversion of glycogen into PHA.

The present disclosure is based, in part, on the finding that infra-red radiation and anaerobic conditions, lead to a selection of a more efficient phototrophic microbial community, for PHA storage.

In some embodiments, step a) is performed in cycles alternating between a cycle in presence of infra-red radiation, and a cycle devoid of light.

The present disclosure is based, in part, on the finding that since glycogen conversion into PHA can occur under dark conditions, the method can be performed under transient illumination conditions, e.g., cycles devoid of light (dark) and light cycles. The technical effect associated is the fact that the cycles devoid of light are PHA productive phases.

In some embodiments, a cycle in the presence of infra-red radiation is performed for at least one period of time from 1 hour (h) to 30 days, preferably from 6 h to 12h. In some embodiments, a cycle devoid of light, is performed for at least one period of time from 1 minute (min) to 48 h, preferably from 4 h to 8 h.

In some embodiments, the electron donor comprises hydrogen. The present disclosure is based, in part, on the finding that hydrogen plays a crucial role as it works as a source of electrons during the light phase.

In some embodiments, the electron donor comprises at least one reduced sulfur compound. The present disclosure is based, in part, on the finding that the at least one reduced sulfur compound plays a crucial role as it works as a source of electrons during the light phase.

The present disclosure is based, in part, on the finding that the use of a reduced sulfur compound allows for reduction in disruptions in the microbial culture since reduced sulfur compounds are toxic for most of bacteria. The technical effect associated, is the selection of relevant microorganisms in the presence of at least a reduced sulfur compound. The use of reduced sulfur compound in each cycle, enables the selection of a mixed microbial culture with the ability to consume carbon dioxide while promoting biomass growth and favoring biopolymer accumulation.

In some embodiments, the at least one reduced sulfur compound is added at a loading rate from 0.01 to 5 mM S d⁻¹. In some embodiments, the at least one reduced sulfur compound is added at a loading rate from 0.05 to 5 mM S d⁻¹. In some embodiments, the at least one reduced sulfur compound is added at a loading rate from 0.1 to 5 mM S d⁻¹. In some embodiments, the at least one reduced sulfur compound is added at a loading rate from 0. 1 to 3 mM S d⁻¹.

In some embodiments, the at least one reduced sulfur compound is added in a concentration from 0.01 to 10 mM. In some embodiments, the at least one reduced sulfur compound is added in a concentration from 0.1 to 10 mM. In some embodiments, the at least one reduced sulfur compound is added in a concentration from 1 to 10 mM. In some embodiments, the at least one reduced sulfur compound is added in a concentration from 2 to 8 mM. In some embodiments, the at least one reduced sulfur compound is added in a concentration of less than 10 mM. In some embodiments, the at least one reduced sulfur compound is added in a concentration of less than 5 mM.

The present disclosure is based, in part, on the finding that anaerobic conditions are fundamental to promote high PHA storage because anaerobic conditions allow for the use of reduced sulfur compounds as electron donor for bacteria (if oxygen was present, it would oxidize the compounds).

The term "reduced sulfur compound" according to the present disclosure, refers to a group of inorganic and organic sulfur compounds that can be used as electron donor by a sulfur oxidizer. Reduced sulfur compound according to the present disclosure, refers to sulfur compounds that can be oxidized by organisms, particularly *Bacteria.* A wide range of sulfur compounds with sulfur having an oxidation state from -2 to +4 can be used as electron donor. Non-limiting examples include sulfide (H₂S), thiosulfate (S₂O₃²⁻), tetrathionate (S₄O₆²⁻), elemental sulfur (S⁰), and sulfite (SO₃²⁻). In some embodiments, a reduced sulfur compound according to the present disclosure is preferably a reduced inorganic sulfur compound.

In some embodiments, reduced sulfur compound comprises elemental sulfur. In some embodiments, reduced sulfur compound comprises a sulfide. Non-limiting examples of sulfide according to the present disclosure include hydrogen sulfide, methane thiol, dimethyl sulfide, carbon disulfide, dimethyl disulfide, and sulfite salts. In some embodiments, at least a sulfide according to the present disclosure is an inorganic sulfide.

In some embodiments, the feedstock is a gas stream, a liquid stream, or both. According to the present disclosure, a gas stream refers to any gas stream comprising CO₂ and H₂S, and a liquid stream refers to any liquid steam rich in carbonate, bicarbonate, reduced sulfur species, or any combination thereof.

Suitable feedstock according to the present disclosure, comprises different streams comprising industrial residues and/or by-products. The technical effect associated is the fact that such feedstocks can be fed to the system (microbial culture comprising at least one phototrophic microorganism, under non-sterile anaerobic conditions and in the presence of infra-red radiation), resulting in the valorization of the disclosed streams.

In some embodiments, the feedstock further comprises at least one nutrient, preferably selected from the list: nitrogen, phosphorus, potassium, magnesium, calcium, iron, sodium, cobalt, zinc, manganese, boron, copper, chlorine, nickel, selenium, iron, molybdenum, bromine, iodine, vitamins, or any combination thereof.

In some embodiments, according to the present disclosure, temperature and pH can be tunned according to the species present in the mixed microbial culture.

In some embodiments, a pH range according to the present disclosure takes into account the balance between a pH for higher solubilization of CO₂ and H₂S (pH > 8), and pH range for anoxygenic photosynthetic bacteria (APB) growth (pH 5 to pH 8).

In some embodiments, the phototrophic mixed microbial culture uses inorganic compounds (including inorganic carbon) from the feed stream (feedstock) to grow and accumulate internal polymers, comprising glycogen and polyhydroxyalkanoates (PHA).

In some embodiments, a method as described herein, further comprises a step of maintaining the at least a first fraction under non-sterile anaerobic conditions, in the presence of infra-red radiation, devoid of light, or alternating between both.

In some embodiments, the method further comprises adding feedstock continuously or discontinuously to the at least a first fraction. The technical effect associated is to further increase PHA content in the at least a first fraction separated from the mixture.

In some embodiments, the method further comprises a step of separating from the at least a first fraction, a solid fraction comprising the PHA. In some embodiments, a method as described herein, further comprises a step of isolating, purifying, or both, the PHA from the solid fraction. Suitable extraction of PHA from a solid fraction are disclosed in the art and would become apparent to those skilled in the art, including physical/mechanical processes, or chemical processes for cell disruption. Depending on the method used for cell disruption, PHA can be recovered for example by evaporation of the solvent or by the addition of a solvent where PHA is poorly soluble subjecting it to successive washes to remove cellular debris. It should be understood and apparent to a skilled person in the art that, depending on the final application of the PHA, the polymer extraction process may contain additional purification steps until a desired degree of purity is achieved.

According to some embodiments, a method as disclosed herein is performed in a bioreactor. In some embodiments, a method as disclosed herein is performed in a system, comprising at least one bioreactor. In some embodiments, the bioreactor is a sequencing batch reactor (SBR). According to some embodiments, step a), step b), or both, of a method as disclosed herein is performed in a bioreactor.

According to the present disclosure, an anaerobic photo-bioreactor is incubated with a phototrophic mixed microbial culture. In some embodiments, the bioreactor is operated in sequential cycles (sequencing batch reactor) under illuminated, non-sterile conditions. In some embodiments a feedstock stream comprising inorganic compounds is fed to the bioreactor.

According to the present disclosure, the type of bioreactor used allows the system to be operated under conditions suitable to prevent oxygen from entering the system. In some embodiments, the configuration of the bioreactor further allows that when illuminated, light is efficiently distributed within the bioreactor, providing adequate illumination to the organisms within it, enabling them to carry out their cellular metabolism. Non-limiting examples of reactors compatible with a method as described herein are panel reactors, tubular reactors or others suitable for operation of photosynthetic organisms.

In some embodiments, between cycles, some of the volume of the reactor is withdrawn. Under these conditions, the mixed culture of phototrophic organisms [phototrophic mixed culture (PMC)] is capable of internally accumulating PHA according to the dominant population of the system. In some embodiments, Glycogen is also accumulated. The conversion of glycogen into PHA is possible due to the anaerobic conditions of the system both during light and dark phase of anoxygenic photosynthesis.

According to some embodiments of the present disclosure, a fraction of the biomass in the reactor (e.g., at least a first fraction) is collected and placed in a parallel bioreactor (accumulation reactor), under conditions as described hereinabove that further increase PHA production.

According to some embodiments, the present disclosure provides a system comprising at least one bioreactor as described herein. In some embodiments, the bioreactor is a sequencing batch reactor (SBR).

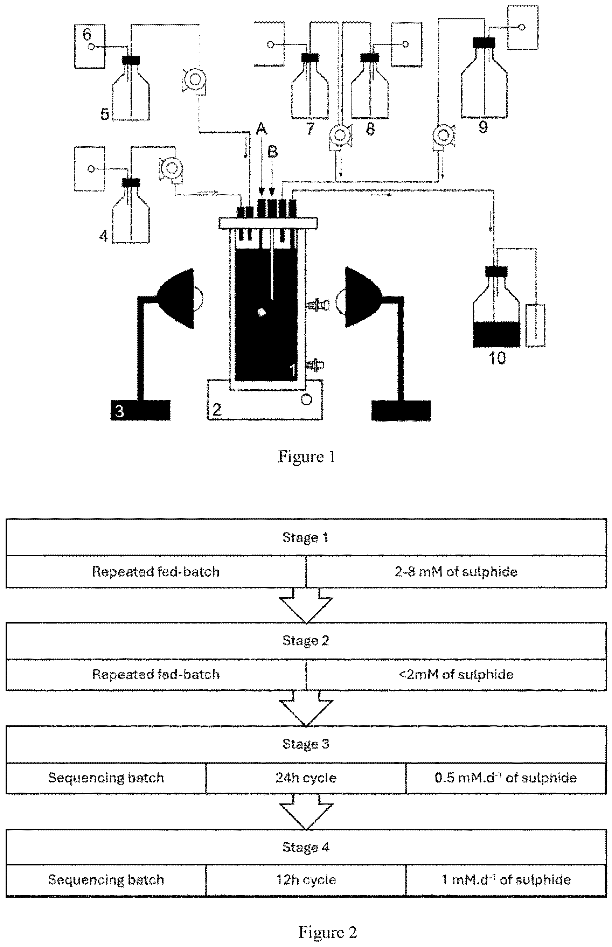

Reference is made to Figure 1 which is a scheme representing an exemplary experimental setup of phototrophic mixed cultures (PMC) selection in a sequencing batch reactor (SBR). In some embodiments, the system comprises a SBR (1), a magnetic stirrer (2), lamps (3), acid and basic solutions for pH control (4 and 5). In some embodiments, the system comprises bags with argon to fill the headspace (6), carbon and sulfide sources (7 and 8), mineral medium (9), purge (10). In some embodiments, the system comprises a headspace outlet (A) and sensor (B) to measure temperature, pH and oxidation reduction potential (ORP).

According to some embodiments, the present disclosure provides a composition comprising polyhydroxyalkanoate (PHA), obtained by the method as described hereinabove.

According to some embodiments, the present disclosure provides a use of the composition as described herein, in animal feed, biocontrol agent, bioplastic, food packaging, drinks packaging, pharmaceuticals packaging, medical devices, tissue engineering, sutures, fibers and clothing, as non-limiting examples.

The term "polyhydroxyalkanoate" (PHA) as used herein, is well known to a person skilled in the art and refers to a class of polyesters of R-hydroxyalkanoic acids. Polyesters are deposited in the form of highly refractive granules in the cells. Depending upon the microorganism and the cultivation conditions, homo- or copolyesters with different hydroxyalkanoic acids are generated.

As used herein the term "about" refers to ± 10 %.

The terms "comprises", "comprising", "includes", "including", "having" and their conjugates mean "including but not limited to".

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable subcombination or as suitable in any other described embodiment of the disclosure.

Various embodiments and aspects of the present disclosure as delineated hereinabove and as claimed in the claims section below find experimental support in the following examples.

### EXAMPLES

Reference is now made to the following examples, which together with the above descriptions illustrate some embodiments of the disclosure in a non-limiting fashion.

### Materials and methods

### Reactor description

The selection of the photosynthetic mixed culture was conducted in a sequencing batch reactor (SBR) with a 2.7 L working volume, continuously stirred with a magnetic stirrer, and maintained at a controlled temperature of 30±1 °C. The reactor pH is maintained at 6.5 ± 0.1, using acid (HCL 2M) and basic solutions (NaOH 3M). It was artificially illuminated by 2 halogen lamps (30W), to achieve a light intensity of 51 W/m² on the internal surface of the reactor wall, which corresponds to 2W/L given the geometry of the bioreactor. The reactor was covered with an UV filter to exclude all wavelengths except for the infra-red light, inhibiting the growth of phototrophic oxygen producers, like microalgae and cyanobacteria (which require visible light). The cover lid of the reactor had entrances that allowed the positioning of a sensor (to measure the temperature, pH, and ORP) and feeding tubes. To maintain constant anaerobic conditions, the reactor is operated completely closed and all feeding solutions are connected to gas-tight bags filled with argon to compensate pressure fluctuations on the system (nitrogen should be avoided to prevent nitrogen fixation activity in the reactor). Every 7 days the reactor is brushed, to prevent excessive biomass attachment on the reactor walls. Immediately after, it is flushed with argon until it reaches an ORP between -300mV and -200mV, to reinstate anaerobic conditions. An exemplary scheme of a reactor according to the present disclosure is shown in Figure 1.

### Operation

The reactor was operated in several conditions. Firstly, it was operated in a repeated fed-batch mode under high concentrations (2-8 mM) of sulfide (Figure 2, stage 1), switching later for lower sulfide concentrations (<2 mM - Figure 2, stage 2). Afterwards, the reactor started being operated under 24h cycles (Figure 2, stage 3) under low sulfide loading rate (SLR) (0.5 mM S d⁻¹) and was later stabilized using 12-hour cycles (Figure 2, stage 4) under increased SLR (1 mM S d⁻¹). In stage 3, the hydraulic retention time (HRT) and the sludge retention time (SRT) were established at 20 days, which was maintained in stage 4. At the beginning of each cycle the reactor was purged trough the top of the reactor, and then fed with mineral medium, carbon source and reduced sulfur species (as electron donor). The inorganic carbon, phosphorus, and nitrogen are maintained at permanent feast conditions, whilst the sulfide is fed in a feast/famine regime in each cycle. The purge is maintained in anaerobic conditions, in a flask previously flushed with argon, and the biomass collected was stored at 4°C.

### Solutions description

Inorganic carbon solution is composed of sodium carbonate (Na₂CO₃); sulfide solution is composed of sodium sulfide (Na₂S·9H₂O); and mineral medium is composed by a mixture of KH₂PO₄, NH₄Cl, MgCl₂.6H₂O, CaCl₂·2H₂O, Vitamin B12, NaCl, EDTA and trace minerals solution (Na₂MoO₄.2H₂O, NiCl₂.6H₂O, CuCl₂.2H₂O, CoCl₂.6H₂O, ZnCl₂, MnCl₂.4H₂O, FeCl₂.4H₂O).

The culture was fed with synthetic medium composed of a carbon medium (C-Feed), sulfide medium (S-feed) and a mineral medium, all fed separately to the reactor. The composition of each medium is provided in Table 1.

**Table 1 - Composition of the carbon, sulfide and mineral medium fed to the selector reactor.**

| | Composition | Concentration |
|---|---|---|
| C-Feed | Na₂CO₃ | 1.08 M |
| S-Feed | Na₂S·9H₂O | 270 mM |
| Mineral medium | KH₂PO₄ | 2.3 mM |
| | NaCl | 4.3 mM |
| | NH₄Cl | 4.7 mM |
| | MgCl₂·6H₂O | 1 mM |
| | CaCl₂·2H₂O | 0.2 mM |
| | Vitamin B12 | 1mL/L |
| | Trace mineral sol. | 1mL/L |
| | EDTA | 1mL/L |

The reactor temperature was controlled at 30°C with a recirculating water bath and pH was controlled at 6.5. The choice of pH is a very important aspect when feeding a reactor with compounds that dissociate in accordance to the system's pH higher solubilization of CO₂ and H₂S can be attained at higher pH values (pH > 8). However, the best pH range for APB growth lies in the acidic range (pH 5 to pH 7) and therefore, a compromise was found by settling the working pH at 6.5, a value that was maintained along the work here presented.

Furthermore, since the development of an APB culture capable of producing PHB using CO₂ as carbon source and sulfide as electron donor requires anaerobic conditions, the reactor was permanently closed. All openings were either blocked with water traps or with gas bags previously filled with argon, which prevented air entrance during reactor sampling and purging.

### EXAMPLE 1

### HIGH SULFIDE VS LOW SULFIDE

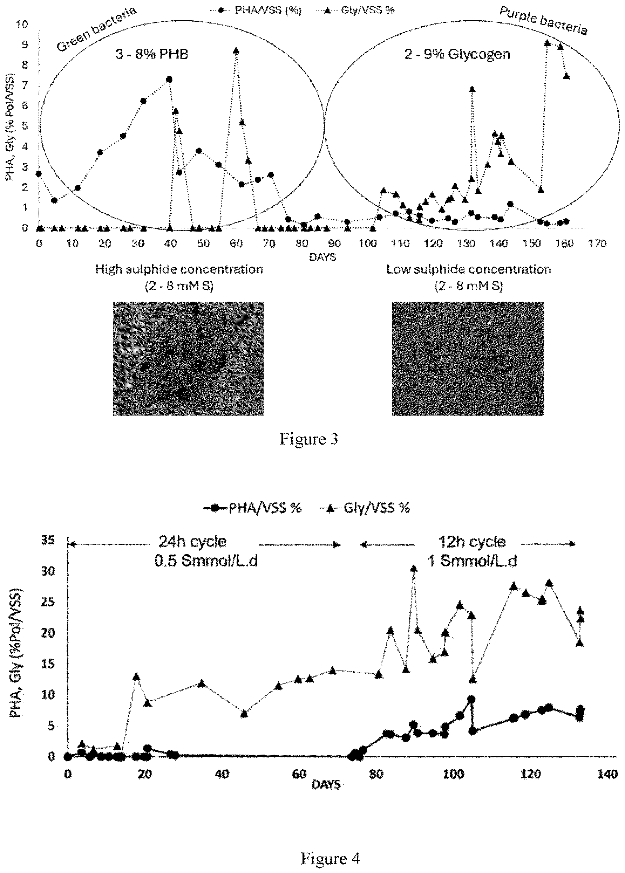

The obtained results in stage 1 showed that the operation of the reactor under high sulfide concentrations favored the photosynthetic green bacteria, that were responsible for the production of biopolymers containing polyhydroxybutirate (PHB) monomers. However, the operation of the reactor was impaired by the gradual appearance of black granules that were attributed to the precipitation of sulfur granules. These granules bestowed an increasingly black color to the reactor, which inhibited light penetration and the photo-microbial culture became unstable (Figure 3).

Switching to lower sulfide concentrations (stage 2) fostered the enrichment in purple bacteria, which accumulate glycogen (Gly) as the primary reserve for carbon and energy. Nevertheless, the operation of the reactor was stable due to the disappearance of the black sulfur precipitates, given that, using this selection strategy, the available sulfide was quickly consumed by the photosynthetic bacterial community (Figure 3). For further microbial enrichment, it was decided to operate the selection reactor in a sequencing batch mode, introducing cycles of feast and famine of sulfides.

### EXAMPLE 2

### 24 HOURS (h) VS 12 h CYCLE

The operation of the reactor in 24h cycles using 0.5 mMS.d⁻¹ (stage 3) did not result in an increase of PHB production, with Gly remaining as the preferred carbon and energy storage (Figure 4). Therefore, it was decided to decrease the cycle length to 12h and increase the sulfur loading rate to 1mMS.d⁻¹ (stage 4), which ultimately promoted higher system stability and culture enrichment in purple bacteria capable of storing 5-10% PHB and 15-25% glycogen. Hence, the selection of a phototrophic mixed culture with PHB accumulation capacity using inorganic carbon as feedstock and sulfide as electron donor, was effectively achieved.

### EXAMPLE 3

### DAILY REACTOR PERFORMANCE

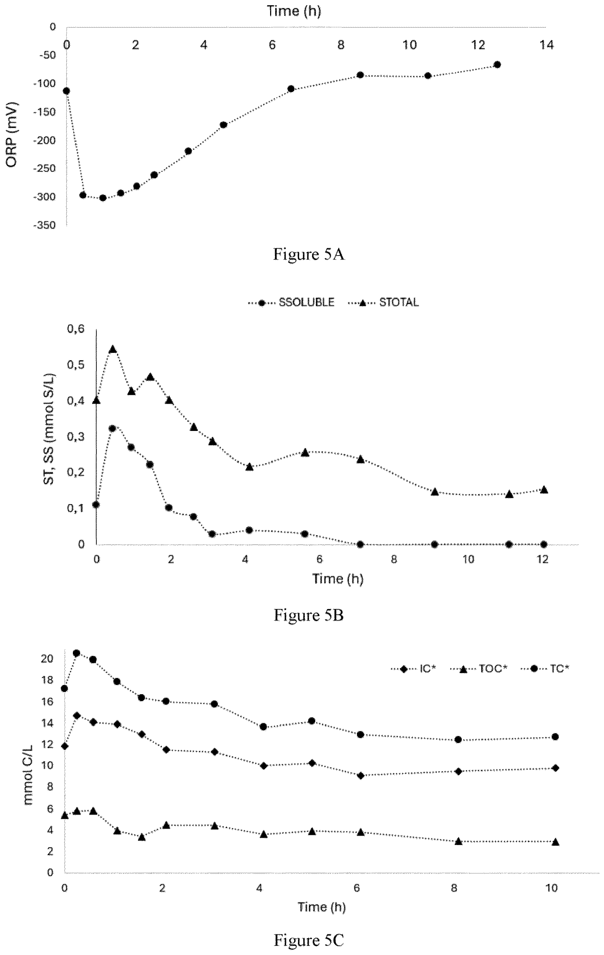

By observing the ORP variation, which is acquired live during reactor operation, and by analyzing the substrate concentration variation overtime, it was possible to determine a direct relationship between an inflection in the ORP curve and the total consumption of the fed substrates (carbon and sulfides). Therefore, the inflection points in the ORP variation started being considered the indicator for the beginning of the sulfide's famine phase. In average, the photo-microbial culture consumes all sulfides in 2-3 hours (Figures 5A-C).

### EXAMPLE 4

### HIGHER LIGHT INTENSITIES

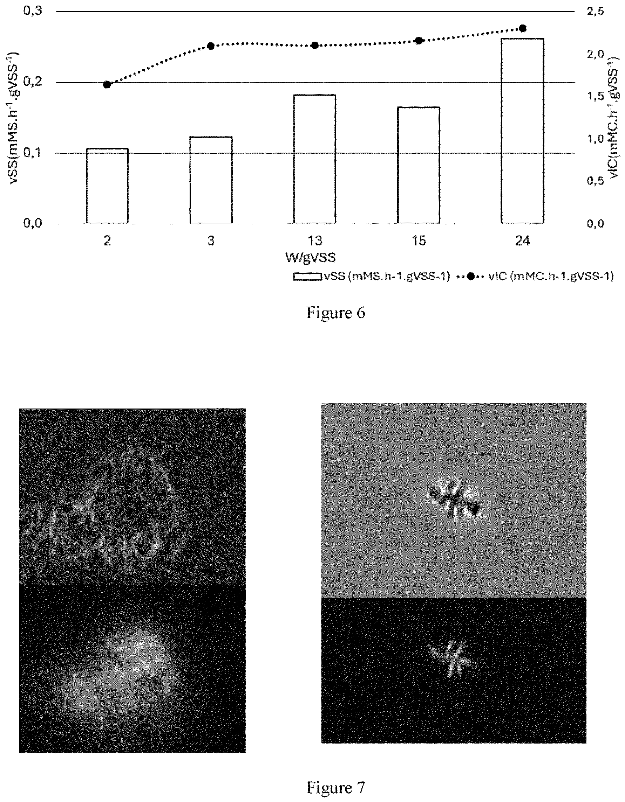

In an attempt to increase the substrate consumption rate, higher light intensities were tested (Figure 6), being observed a slight increase in consumption rates for both sulfides and inorganic carbon. However, the light intensity did not impact biopolymer production, with the Gly remaining at 15% (%VSS) and PHB remaining at 5% (%VSS).

### EXAMPLE 5

### DARK PHASE TESTS

Given that Gly remained as the primary biopolymer accumulated by this photosynthetic microbial culture, tests were made to assess the microbial behavior in the absence of light. In fact, it was observed that some bacteria that usually do not contribute to PHB accumulation started to present fluorescence with the Nile Blue staining, after 6h in the dark (Figure 7). Effectively, PHA accumulation was favored in dark conditions by the transformation of previously accumulated Gly (during light availability) into polyhydroxybutirate-hydroxyvalerate (HB-HV) monomers. Specifically, a decrease from 15% Gly (%VSS) to 2% Gly (%VSS) translated into the production of 13% HB-HV (%VSS) in dark conditions.

Although the disclosure has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

## Claims

1. Method for production of polyhydroxyalkanoate (PHA), the method comprising:
a) adding to a microbial culture comprising at least one phototrophic microorganism, a feedstock comprising at least one inorganic carbon compound and at least one electron donor, wherein said adding is performed continuously or discontinuously, under non-sterile anaerobic conditions and in the presence of infra-red radiation, thereby obtaining a mixture comprising PHA; and
b) separating at least a first fraction from said mixture.

2. Method according to claim 1, wherein said step a) is performed in cycles alternating between a cycle in presence of infra-red radiation, preferably for at least one period of time from 1 hour (h) to 30 days, more preferably from 6 h to 12 h, and a cycle devoid of light, preferably for at least one period of time from 1minute (min) to 48 h, more preferably from 4 h to 8 h.

3. Method according to any one of claims 1 to 2, wherein said electron donor comprises at least one reduced sulfur compound, preferably at least one sulfide.

4. Method according to any one of claims 1 to 3, wherein said electron donor comprises hydrogen.

5. Method according to any one of claims 1 to 4, wherein said feedstock is a gas stream, a liquid stream, or both.

6. Method according to any one of claims 1 to 5, wherein said inorganic carbon compound comprises carbon dioxide, carbonic acid, carbonate, bicarbonate salt or any combination thereof.

7. Method according to any one of claims 1 to 6, wherein said feedstock further comprises at least one nutrient, preferably selected from the list: nitrogen, phosphorus, potassium, magnesium, calcium, iron, sodium, cobalt, zinc, manganese, boron, copper, chlorine, nickel, selenium, iron, molybdenum, bromine, iodine, vitamins, or any combination thereof.

8. Method according to any one of claims 1 to 7, wherein said microbial culture comprises bacteria, preferably an anoxygenic phototrophic bacteria.

9. Method according to any one of claims 1 to 8, wherein said adding comprises permanent feed and/or intermittent feed of said at least one inorganic carbon compound, at least one electron donor, or both.

10. Method according to any one of claims 1 to 9, further comprising a step of maintaining said at least a first fraction under non-sterile anaerobic conditions, in the presence of infra-red radiation, devoid of light, or alternating between both.

11. Method according to claim 10, further comprising adding feedstock continuously or discontinuously to said first fraction.

12. Method according to any one of claims 1 to 11, further comprising a step of separating from said first fraction, a solid fraction comprising said PHA.

13. Method according to claim 12, further comprising isolating, purifying, or both, said PHA from said solid fraction.

14. Composition comprising polyhydroxyalkanoate (PHA), obtained by the method of any one of claims 1 to 13.

15. Use of the composition according to claim 14, in animal feed, biocontrol agent, bioplastic, food packaging, drinks packaging, pharmaceuticals packaging, medical devices, tissue engineering, sutures, fibers and clothing.
